# EUROPÄISCHE PATENTANMELDUNG

(11) **EP 3 431 164 A1**
(43) Veröffentlichungstag der Anmeldung: **23.01.2019**
(21) Anmeldenummer: 18170517.9
(22) Anmeldetag: 03.05.2018
(51) Int. Cl.: B01D 46/00, B01D 46/42, B01D 53/00, B01D 53/88, B60H 3/06

(54) **LUFTREINIGUNGSVORRICHTUNG**

(30) Priorität: 19.07.2017 DE 102017116307
(71) Anmelder: Dr. Schneider Kunststoffwerke GmbH, 96317 Kronach-Neuses (DE)
(72) Erfinder: FIEDLER, Jochen, 96317 Kronach (DE)

(57) **Zusammenfassung**

Die vorliegende Erfindung betrifft Luftreinigungseinheit mit einem Gehäuse, das mindestens eine Eingangsöffnung zur Zuführung eines Luftstroms und mindestens eine Ausgangsöffnung zur Ausleitung des über die Eingangsöffnung zugeführten Luftstroms besitzt. Im Gehäuse ist ein Ventilator, mindestens eine Luftreinigungseinheit und mindestens eine Beleuchtungseinheit angeordnet sind, wobei die mindestens eine Luftreinigungseinheit und die mindestens eine Beleuchtungseinheit einander gegenüberliegend im Gehäuse angeordnet sind. Die mindestens eine Luftreinigungseinheit besitzt mindestens einen mit Aktivkohle durchsetzten Bereich und einen photokatalytisch wirksamen Bereich. Die mindestens eine Eingangsöffnung zur Zuführung eines Luftstroms und mindestens eine Ausgangsöffnung zur Ausleitung des über die Eingangsöffnung zugeführten Luftstroms sind jeweils mit einer Verschlussklappe schliessbar, so dass eine Selbstreinigung der Luftreinigungseinheit durchgeführt werden kann.

## Beschreibung

Die vorliegende Einrichtung dient zur Reinigung von Luft, insbesondere zur Reinigung von Luft in Kraftfahrzeuginnenräumen.

Aus dem Stand der Technik sind verschiedene Vorrichtungen zur Luftreinigung in Kraftfahrzeugen bekannt. Diese sind zumeist derart ausgebildet und angeordnet, dass Luft kontinuierlich aus dem Fahrzeuginnenraum abgesaugt, über einen Filter geführt und dann dem Fahrzeuginnenraum wieder zugeführt wird.

So ist aus der DE 10 2014 012 870 A1 ein Luftreiniger bekannt. Dieser Luftreiniger verwendet ultraviolette Strahlen zur Luftreinigung. Der Luftreiniger weist ein Gehäuse mit einem Gehäuseeinlass und einem -auslass, ein Gebläse, das im Gehäuse, angrenzend an den Lufteinlass, angeordnet ist, eine ultraviolette Leuchtdiodeneinheit und eine Filtereinheit auf. Die Filtereinheit ist im Gehäuse oberhalb des Gebläses, entlang eines Strömungsweges der Luft, angeordnet. Es ist außerdem eine Strömungsanordnung vorhanden, die im Gehäuse, zwischen dem Gebläse und der Filtereinheit, angeordnet ist. Dabei steuert die Strömungssteueranordnung den Luftstrom entlang des Strömungswegs der Luft zwischen dem Auslass des Gebläses und der Filtereinheit. Zur Reinigung der Luft kommen hierbei photokatalytisch wirksame, ultraviolette Leuchtdioden zum Einsatz.

Aus der DE 20 2007 019 288 U1 ist ein System zur Luftreinigung unter Verwendung von Ozon und einem keramischen, porösen Katalysator offenbart. Das System weist ein Gehäuse, mindestens einen Einlass und einen Auslass, mindestens eine Photonenquelle, einen keramischen Kern und ein Fluidstromerzeugungsgerät auf, wobei die Photonenquelle stromaufwärts von dem keramischen Kern angeordnet ist.

Aus der EP 0 707 989 A1 ist eine Vorrichtung zur Reinigung von Luft bekannt. Eine Reinigung erfolgt in der Weise, dass Außenluft über ein Gehäuse und ein darin angeordnetes Gebläse angesaugt, in mehreren Reinigungsstufen gereinigt und dann dem Innenraum des Fahrzeugs zugeführt wird. Das Gebläse bzw. der Antrieb des Gebläses wird über Solarzellen, die am Fahrzeug angeordnet sind und als Energiequellen dienen, gespeist.

Aus der EP 2 181 720 A1 ist eine Vorrichtung zur photokatalytischen Dekontamination eines Luftstroms bekannt. Die Vorrichtung umfasst ein Gehäuse mit mindestens einem Einlass und einem Auslass, mindestens eine Lichtquelle und mindestens einen Träger. Der Träger umfasst einen Photokatalysator, wobei die Lichtquelle zumindest eine Oberfläche des Trägers beleuchtet um den Photokatalysator zu aktivieren. Der zu dekontaminierende Luftstrom wird vom Einlass über den Photokatalysator zum Auslass geleitet. Der Träger besteht aus einer festen Metallverbindung, die antimykotisch und bakteriostatisch ist. Der Luftstrom im Gehäuse wird über eine Zirkulationseinrichtung entlang des Trägers geführt.

Aus der US 5 919 422 A ist eine Luftreinigungsvorrichtung für ein Kraftfahrzeug bekannt. Diese Luftreinigungsvorrichtung besteht aus einem Gehäuse in dem ein phototakatytischer Prozess erzeugbar ist, mittels dessen die zugeführte Luft gereinigt wird.

Nachteilig bei dem vorgenannten Stand der Technik ist, dass die Reinigungswirkung und insbesondere die Beseitigung von Schadpartikeln oder Geruchspartikeln aus der zu reinigenden Luft nicht ausreichend und nicht über einen längeren Zeitraum ohne Wartungseingriffe möglich sind. Dies ist zum einen dadurch bedingt, dass zumeist ein mechanischer Filter verwendet wird, der nach relativ kurzer Zeit bereits eine solche Menge an Partikeln aufgenommen hat, dass er getauscht werden muss, um die ursprüngliche Reinigungsleistung beizubehalten. Beim Einsatz von Filtern mit Aktivkohle und photokatalytischen Bereichen müssen diese nach einem geraumen Einsatzzeitraum ausgetauscht werden, da die Filter mit Aktivkohle zum einen Feuchtigkeit binden und dadurch ihren Reinigungseffekt verlieren, da die Filter gesättigt werden, zum andern die photokatalytischen Bereichen sich zusetzen und dadurch Ihre Wirksamkeit verlieren. Erfolgt kein regelmäßiger Austausch, trägt ein solcher Filter mehr zur Verunreinigung der Luft bei, als dass er diese reinigt. Es dürfte den meisten Benutzern von Klimaanlagen bekannt sein, dass ein verschmutzter Filter erheblich zur Geruchsbelästigung in einem Kraftfahrzeug beiträgt.

Weiterhin ist nachteilig, dass ein Filterwechsel mit relativ hohen Kosten verbunden ist, da ein solcher Wechsel zumeist nur in einer Fachwerkstatt vorgenommen werden kann.

Im Weiteren ist zu berücksichtigen, dass vor allem neue Fahrzeuge über einen bestimmten Zeitraum flüchtige organische Verbindungen (VOC) und andere gesundheitsschädlich Substanzen abgeben. Um diese möglichst umgehend und ohne Belastung für die Insassen eines Fahrzeugs zu beseitigen, sind aus dem Stand der Technik bisher keine Systeme bekannt. Zwar sind im Stand der Technik Filtersysteme, wie eingangs beschrieben, vorhanden, welche auch solche Verbindungen herausfiltern können, jedoch ist der Zeitraum, über welchen sich diese Verbindung im Fahrzeug halten, verhältnismäßig lang.

Es ist daher Aufgabe der vorliegenden Erfindung, die Nachteile des Standes der Technik zu beheben und eine Luftreinigungsvorrichtung anzugeben, welche flüchtige organische Stoffe und gesundheitsschädlich Substanzen sowie andere Luftverunreinigungen gezielt in der Luft im Fahrzeuginnenraum reduziert und zusätzlich auch unangenehme Gerüche vermeidet und beseitigt und eine Selbstreinigungsfunktion besitzt.

Diese Aufgabe wird durch eine Luftreinigungsvorrichtung mit den in Anspruch 1 angegebenen technischen Merkmalen gelöst. Vorteilhafte Weiterbildungen sind in den Unteransprüchen, der weiteren Beschreibung und insbesondere der Beschreibung anhand konkreter Ausführungsbeispiele, angegeben.

Die vorliegende Erfindung betrifft eine Luftreinigungseinheit mit einem Gehäuse, das mindestens eine Eingangsöffnung zur Zuführung eines Luftstroms und mindestens eine Ausgangsöffnung zur Ausleitung des über die Eingangsöffnung zugeführten Luftstroms besitzt. Im Gehäuse sind mindestens eine Luftreinigungseinheit und mindestens eine Beleuchtungseinheit angeordnet. Die mindestens eine Luftreinigungseinheit nimmt im Wesentlichen die Form des Querschnitts des Gehäuses auf. Vorteilhaft ist die Querschnittsfläche nahezu ausfüllend im Gehäuse angeordnet. Im Gehäuse ist die mindestens eine Beleuchtungseinheit der mindestens einen Luftreinigungseinheit gegenüberliegend angeordnet. Die mindestens eine Luftreinigungseinheit ist luftdurchlässig ausgestaltet und weist auf der der Beleuchtungseinheit zugewandten Seite mindestens einen photokatalytisch wirksamen Oberflächenbereich bzw. Flächenbereich auf. Außerdem weist sie mindestens einen mit Aktivkohle durchsetzten bzw. versetzten Bereich, der zumindest partiell von einem Vlies umgeben ist, auf, wobei auf der der Beleuchtungseinheit zugewandten Seite der mindestens einen Luftreinigungseinheit mindestens ein photokatalytisch wirksamer Oberflächenbereich im oder am Vlies vorhanden ist und der Luftstrom im Gehäuse zumindest partiell den mindestens einen photokatalytisch wirksamen Oberflächenbereich bzw. Flächenbereich und den mit Aktivkohle versetzen Flächenbereich umströmt bzw. durchströmt. Der mindestens eine photokatalytisch wirksame Oberflächenbereich bzw. Flächenbereich der mindestens einen Luftreinigungseinheit ist von der mindestens einen Beleuchtungseinheit mit Licht anstrahlbar. Die mindestens eine Eingangsöffnung zur Zuführung des Luftstroms und die mindestens eine Ausgangsöffnung zur Ausleitung des über die Eingangsöffnung zugeführten Luftstroms sind mittels jeweils einer Verschlussklappe schließbar. An Stelle der Verschlussklappe kann auch eine Membran eingesetzt werden. Im geschlossenen Zustand der beiden Verschlussklappen wird im Gehäuse ein Raumvolumenbereich abgegrenzt. Es ist ein Ventilator im Gehäuse im abgegrenzten Volumenbereich angeordnet, der im Raumvolumenbereich des Gehäuses einen Luststrom erzeugt, wobei der erzeugte Luftstrom den mit Aktivkohle durchsetzten Bereich durchströmt, und die mindestens eine Beleuchtungseinheit den abgegrenzten Raumvolumenbereich des Gehäuses und damit den Luftstrom und den mit Aktivkohle durchsetzten Bereich erhitzt und somit eine Selbstreinigung des mit Aktivkohle durchsetzten Bereichs und/oder des Vlieses und/oder des photokatalytisch wirksamen Oberflächenbereich erfolgt.
Durch die Aufheizung des über die Verschlussklappen begrenzten Raumvolumenbereiches des Gehäuses über die aktivierte Beleuchtungseinheit wird in der Aktivkohle gebundenes Wasser ausgegast. Dadurch entsteht bei gleich bleibender Temperatur eine erhöhte relative Luftfeuchte. Es wird die Temperatur im Gerät soweit erhöht, dass die relative Luftfeuchte niedriger ist als die initiale relative Luftfeuchte. Die hierdurch verringerte relative Luftfeuchtigkeit trägt zur Verbesserung der photokatalytischen Reinigungswirkung im Rahmen der Selbstreinigung bei. Zugleich werden durch die Temperaturerhöhung, die UV-Bestrahlung und den über den Ventilator erzeugten Luftstrom der Luftstrom und die gesamte Luftreinigungseinheit erhitzt. Hierdurch wird eine Schadstoffdesorption aus der Luftreinigungseinheit erreicht und die Aktivkohle getrocknet. Die desorbierten Gase werden mithilfe der Photokatalyseeinheit aufgereinigt. Nach einem vordefinierbaren Zeitraum werden die Verschlussklappen geöffnet und die aufgreinigten Stoffe entweichen. Die Luftreinigungsvorrichtung hat den Selbstreinigungsprozess abgeschlossen.

Es ist in einer vorteilhaften alternativen Ausgestaltung der Erfidnung vorgesehen, dass die Luft mit den desorbierten Stoffen über einen Bypass außerhalb des Innenraums des Fahrzeuges ausleitbar ist.

In einer vorteilhaften Ausgestaltung ist auf der der Beleuchtungseinheit abgewandte Seite mindestens ein mit Aktivkohle versetzter bzw. durchsetzter Flächenbereich vorhanden. Der Luftstrom im Gehäuse umströmt dann zumindest partiell den mindestens einen photokatalytisch wirksamen Oberflächenbereich und durchströmt den mit Aktivkohle versetzten Flächenbereich.

In einer weiteren vorteilhaften Ausgestaltung der Erfindung ist das Vlies mit Aktivkohle versetzt bzw. durchsetzt. Außerdem sind im Vlies und/oder im mit Aktivkohle befüllten Flächenbereich Heizdrähte angeordnet, die bei der Selbstreinigung aktiviert sind und damit das Aufheizen beschleunigen und eine höhere Temperatur bei der Selbstreinigung erzielen lassen.

In einer vorteilhaften Ausgestaltung der Erfindung nach Patentanspruch 2 ist vorgesehen, dass der mindestens eine photokatalytisch wirksame Oberflächenbereich zumindest teilweise mit einem Metalloxid oder einem Mischoxid beschichtet oder mit einem Metalloxid oder einem Mischoxid - versetzt oder durchsetzt ist und dass zusätzlich Heizdrähte integriert sind, die bei der Selbstreinigung zur schnellen Erhitzung beitragen und/oder wobei das Metalloxid ein Oxid aus einem Übergangsmetall ist.

Unter einem Mischoxid, kurz auch mit MOX bzw. MOx bezeichnet, versteht man einen Stoff, der sich aus mehreren Oxiden zusammensetzt. Es gibt eine Vielzahl von Mischoxiden. Unter einem Metalloxid versteht man eine Verbindung zwischen einem Metall und Sauerstoff.

In einer vorteilhaften Ausgestaltung der Erfindung nach Patentanspruch 3 ist vorgesehen, dass das Metalloxid ein Oxid aus einem Übergangsmetall ist.
Als Übergangselemente werden die chemischen Elemente mit den Ordnungszahlen von 21 bis 30, 39 bis 48, 57 bis 80 und 89 bis 112 bezeichnet. Da diese Elemente alle Metalle sind, werden diese Elemente auch als Übergangsmetalle bezeichnet. Dieser Begriff Übergangsmetalle ergibt sich auf Grund der Position dieser Elemente im Periodensystem begründet, da sich dort der Übergang durch die aufeinanderfolgende Zunahme von Elektronen in den d-Atomorbitalen entlang jeder Periode zeigt. Übergangselemente werden von der IUPAC definiert als Elemente, die eine unvollständige d-Unterschale besitzen oder Ionen mit einer unvollständigen d-Unterschale ausbilden.

Das Metalloxid oder Mischoxid besteht aus CuO, Co₃O₄, CoOₓ, NiO, MnOₓ, MnO₂, MoO₃, ZnO, Fe₂O₃, WO₃, CeO₂, TiO₂, Al₂O₃, V₂O₃, ZrO₂, HfO₂, Dy₂O₃, Cr₂O₃ LiNbO₃ und/oder Nb₂O₅.

Bei photochemischen Katalysatoren, wie im vorliegenden Fall, können einzelne Oxide von Übergangsmetallen in Form von Mischoxiden zum Einsatz kommen. Diese Katalysatoren mit Oxiden von Übergangsmetallen sind wirksam und erwirken eine vollständige und/oder selektive Oxidation von flüchtigen organischen Verbindungen.

Die katalytische Oxidation von flüchtigen organischen Verbindungen mittels Katalysatoren auf Basis von Edelmetallen ist für deren Struktur und deren strukturellen Eigenschaften teilweise schädlich. Die Wirkung von Pt-Partikelgröße (Pt = Platin) auf die katalytische Oxidation von verschiedenen Kohlenwasserstoffen hat gezeigt, dass im Allgemeinen größere Pt-Teilchen aktiver sind als kleinere PT-Teilchen. Eine geringere Auswirkungen auf die katalytische Wirksamkeit von Pt-Katalysatoren ist durch Faktoren wie die Art des Trägers (Aluminiumoxid oder Siliciumdioxid) ausgeübt wird, die Porosität und Säure-Base-Eigenschaften des Trägers. Die Zugabe von Co₃O₄, CeO₂, La³⁺ / Bi³⁺ Promotoren zu CeO₂-ZrO₂ führt zu einer Erhöhung der Aktivität und Wärmestabilität von Katalysatoren mit Pt und Pd (Palladium) auf Basis von Aluminiumoxid, sowie zur Reduktion von flüchtigen organischen Verbindungen.
Edelmetall-Katalysatoren, wie Pt und Pd (Palladium), zeigen eine gute Wirkung bei niedrigen Temperaturen in vollständige Oxidation von flüchtigen organischen Verbindungen. Die Anwendung derartiger Katalysatoren zur Katalyse von flüchtigen organischen Verbindungen ist zum Einsatz im Innenraum von Fahrzeugen begrenzten aufgrund der hohen Kosten und der technisch bedingten Gefahr von Vergiftungen auf Grund der möglichen Bildung von Chlor und Chlorderivaten. Ceriumoxid hingegen ist sehr aktiv auf Grund seiner Fähigkeit von Sauerstoffanreicherung. Oxidierung von flüchtigen organischen Verbindungen zu CeO₂ basiert auf dem Redox-Mechanismus. Die Modifikation von CeO₂ mit anderen Metalloxiden, z.B. durch teilweisen Ersatz von Ce⁴⁺-Ionen durch Zr⁴⁺-Ionen im Gitternetz (gemischte Ce-Zr-Oxide), kann die Sauerstoffkapazität und thermische Beständigkeit des Katalysators, sowie Erhöhung der katalytischen Aktivität bei niedrigen Temperaturen, verbessern. Ein Vorteil der Katalysatoren auf Manganbasis ist deren hohe Aktivität für alle Oxidationsreaktionen im Zusammenhang mit damit entstehenden niedrigen Kosten und geringen Toxizität.
Auch Katalysatoren mit der Perowskit-Struktur können zum Einsatz kommen.
Sehr gute katalytische Eigenschaften werden auch von Perowskit-Strukturen mit deren allgemeine Formel ABO₃ erzielt. Bei dieser Formel ist A das Element der Seltenen Erden, B ein Übergangsmetall ist.

In einer besonders gut zu realisierenden Ausgestaltung der Erfindung ist der mindestens eine photokatalytisch wirksame Oberflächenbereich zumindest teilweise mit Titandioxid - Ti02 - beschichtet oder mit Titandioxidionen - Ti02-Ionen - versetzt oder durchsetzt. Es kann aber auch eine jeder andere der vorgenannten Verbindungen, die photokatalytisch wirksam ist, zum Einsatz kommen.

In einer vorteilhaften Ausgestaltung der Erfindung nach Patentanspruch 4 ist vorgesehen, dass der photokatalytisch wirksame Oberflächenbereich derart abgeordnet ist, dass dieser nach unten in Richtung des Bodens des Gehäuses (2) geneigt ist. Damit kann sich der photokatalytisch wirksamen Oberflächenbereich nicht zusetzen, da Staub etc. von diesem abfällt.

In einer vorteilhaften Ausgestaltung der Erfindung nach Patentanspruch 5 ist vorgesehen, dass die mindestens eine Luftreinigungseinheit an einer der Innenwände des Gehäuses angeordnet oder fixierbar ist. Durch die Anordnung bzw. Fixierung der mindestens einen Luftreinigungseinheit an einer der Innenwände des Gehäuses kann eine gute Befestigung erfolgen, zugleich kann der Luftstrom im Gehäuse so gelenkt werden, dass er optimal auf die mindestens eine Luftreinigungseinheit abgestimmt ist. Bei einer Anordnung in der Mitte des Gehäuses kann die mindestens eine Luftreinigungseinheit direkt vom in das Gehäuse einströmenden Luftstrom umgeben werden und es wird eine gute Reinigungsleistung erzielt.

In einer vorteilhaften Ausgestaltung der Erfindung nach Patentanspruch 6 ist vorgesehen, dass die mindestens eine Beleuchtungseinheit im Gehäuse zwischen dem mindestens einen photokatalytisch wirksamen Oberflächenbereich und der Luftaustrittsöffnung angeordnet ist, und wobei nur die Innenwände des Gehäuses im Bereich zwischen der mindestens einen Luftreinigungseinheit und der mindestens eine Beleuchtungseinheit mit einem lichtreflektierenden Material, insbesondere einem UV-Licht reflektierenden Material, beschichtet sind.
Die Beleuchtungseinheit ist erforderlich, da nur durch auf die mindestens eine Luftreinigungseinheit bzw. deren photokatalytisch wirksame Oberflächenbereichen auftreffendes Licht, vorzugsweise UV-Licht, der photokatalytische Reinigungsprozess in Gang gesetzt wird. Daher ist durch die Anordnung der mindestens einen Beleuchtungseinheit gegenüber der mindestens einen Luftreinigungseinheit eine gute Beleuchtung und damit Zuführung von UV-Licht möglich. Durch die zusätzliche Einbringung von UV-Licht reflektierenden Material wird die UV-Beleuchtung intensiviert.

In einer vorteilhaften Ausgestaltung der Erfindung ist vorgesehen, dass die mindestens eine Beleuchtungseinheit aus mindestens einer Leuchtdiode besteht, die UV-Licht emittiert. Je nach Dotierung des Photokatalysators kann eine Aktivierung auch mit sichtbarem Licht erfolgen.

In einer vorteilhaften Ausgestaltung der Erfindung nach Patentanspruch 7 ist vorgesehen, dass vor der mindestens einen Beleuchtungseinheit Linsen angeordnet sind, die das von der mindestens einen Beleuchtungseinheit emittierte Licht auf die mindestens eine Luftreinigungseinheit fokussieren oder hinter und/oder neben der mindestens einen Beleuchtungseinheit Lichtreflektoren oder lichtreflektierende Bereiche angeordnet sind, welche das von der mindestens einen Beleuchtungseinheit emittierte Licht in Richtung der mindestens einen Luftreinigungseinheit reflektieren. Durch die Anordnung der Linsen wird eine besonders gute Fokussierung des emittierten Lichts auf die mindestens eine Luftreinigungseinheit erreicht. Durch die Anordnung von Lichtreflektoren oder lichtreflektierenden Bereichen wird erreicht, dass auch Licht, das nicht direkt auf die mindestens eine Luftreinigungseinheit gerichtet wird, dennoch zumindest teilweise zur Luftreinigungseinheit geleitet wird.

In einer vorteilhaften Ausgestaltung der Erfindung nach Patentanspruch 8 ist vorgesehen, dass die Innenwände des Gehäuses weiß lackiert oder mit einem lichtreflektierenden Material beschichtet sind. Dadurch wird erreicht, dass das von der mindestens einen Beleuchtungseinheit emittierte Licht nicht vom Gehäuse absorbiert wird, sondern reflektiert und damit zusätzlich auf die bzw. in Richtung der mindestens einen Luftreinigungseinheit geleitet wird.

In einer vorteilhaften Ausgestaltung der Erfindung nach Patentanspruch 9 ist vorgesehen, dass im Gehäuse Luftleitelemente angeordnet sind, welche den Luftstrom diffus im Gehäuse ablenken, sodass der Luftstrom nahezu gleichmäßig auf die gesamte Oberfläche des Vlieses der mindestens einen Luftreinigungseinheit trifft und diese durchströmt oder der Luftstrom größtenteils auf den mindestens einen photokatalytisch wirksamen Oberflächenbereich gerichtet ist und diesen umströmt bzw. durchströmt. Zusätzlich entstehen dadurch gewollte Verwirbelungen der Luft. Damit wird eine besonders hohe Reinigungsleistung erzielt.

In einer vorteilhaften Ausgestaltung der Erfindung nach Patentanspruch 10 ist vorgesehen, dass die mindestens eine Luftreinigungseinheit aus einem Rahmen aus Kunststoff oder Metall besteht, welcher vom Vlies zumindest an einer Seite umschlossen ist und hinter dem Vlies ein Aufnahmebereich für Granulatstoffe vorhanden ist, wobei dieser Aufnahmebereich mit Aktivkohle oder einem Gemisch aus Aktivkohle und/oder mit Titandioxid - TiO2 - oder Metalloxid oder Mischoxid von CuO, Co₃O₄, CoOₓ, NiO, MnOₓ, MnO₂, MoO₃, ZnO, Fe₂O₃, WO₃, CeO₂, TiO₂, Al₂O₃, V₂O₃, ZrO₂, HfO₂, Dy₂O₃, Cr₂O₃ und/oder Nb₂O₅ beschichtetem oder mit Titandioxidionen - TiO2-Ionen - und/oder Metalloxid-Ionen oder Mischoxid-Ionen von CuO, Co₃O₄, CoOₓ, NiO, MnOₓ, MnO₂, MoO₃, ZnO, Fe₂O₃, WO₃, CeO₂, TiO₂, Al₂O₃, V₂O₃, ZrO₂, HfO₂, Dy₂O₃, Cr₂O₃ und/oder Nb₂O₅ versetztem Granulat befüllt ist.
Damit kann durch den photokatalytischen Prozess, der bei lichtauftreffen auf Titandioxid bzw. einem der vorgenannten Materialien ausgelöst wird, auch der Aktivkohlebereich gereinigt werden, sodass der Filter längere Zeit seine vollständige Reinigungsleistung erreicht. Außerdem können dadurch Verunreinigungen, die am Vlies anhaften, entfernt bzw. beseitigt werden.

In einer vorteilhaften Ausgestaltung der Erfindung nach Patentanspruch 11 ist vorgesehen, dass zumindest partiell Fäden oder Fasern in das Vlies eingearbeitet oder eingebracht sind, die mit Titandioxidionen - TiO2-Ionen oder Metalloxid-Ionen oder Mischoxid-Ionen von CuO, Co₃O₄, CoOₓ, NiO, MnOₓ, MnO₂, MoO₃, ZnO, Fe₂O₃, WO₃, CeO₂, TiO₂, Al₂O₃, V₂O₃, ZrO₂, HfO₂, Dy₂O₃, Cr₂O₃ und/oder Nb₂O₅ dotiert sind, welche den mindestens einen photokatalytisch wirksamen Oberflächenbereich (53) ausbildet oder dass Bereiche (53) des Vlieses (51) mit Titandioxidionen - TiO2-Ionen oder Metalloxid-Ionen oder Mischoxid-Ionen von CuO, Co₃O₄, CoOₓ, NiO, MnOₓ, MnO₂, MoO₃, ZnO, Fe₂O₃, WO₃, CeO₂, TiO₂, Al₂O₃, V₂O₃, ZrO₂, HfO₂, Dy₂O₃, Cr₂O₃ und/oder Nb₂O₅ dotiert sind. Es wird durch den photokatalytischen Prozess, der bei lichtauftreffen auf Titandioxid bzw. einem der vorgenannten Materialien aktiviert wird, nicht nur die vorbeiströmende Luft gereinigt, sondern zugleich auch der Aktivkohlebereich und am Vlies anhaftende Verunreinigungen werden entfernt, sodass der Filter längere Zeit seine vollständige Reinigungsleistung erreicht.

In einer vorteilhaften Ausgestaltung der Erfindung nach Patentanspruch 12 ist vorgesehen, dass die Oberfläche der mindestens einen Luftreinigungseinheit mit dem Vlies zumindest an der den mindestens einen photokatalytisch wirksamen Oberflächenbereich aufweisenden Seite wellenförmig ausgestaltet ist oder kegel-, falten-, zylinder-, kegelstumpf-, pyramidenstumpf-, kugel- oder halbkugelgeometrische Ausformungen besitzt. Durch diese Formgebung kann die für die Luftreinigung wirksame Oberfläche der mindestens einen Luftreinigungseinheit vergrößert werden. Die konkrete Formgestaltung der Oberfläche führt nur zu einer geringen Erhöhung des Luftwiderstandes, der dem Luftstrom im Gehäuse entgegenwirkt.

In einer vorteilhaften Ausgestaltung der Erfindung nach Patentanspruch 13 ist vorgesehen, dass die mindestens eine Beleuchtungseinheit in ihrer Lichtintensität steuerbar ist, und bei der Selbstreinigung mit maximaler Intensität angesteuert ist.

Es kann zusätzlich ein Luftgütemesssensor, der im Lufteinlasskanal oder am Luftauslasskanal angeordnet ist, vorhanden sein, der die Luftgüte ermittelt und eine Steuereinheit anhand der vom Luftgütemesssensor ermittelten Luftgüte die Lichtintensität der mindestens einen Beleuchtungseinheit steuert. Damit kann zum einen die elektrische Leistungsaufnahme bedarfsgerecht gesteuert werden, zugleich kann vermieden werden, dass die mindestens eine Beleuchtungseinheit kontinuierlich, auch wenn nicht erforderlich, mit maximaler elektrischer Last betrieben wird. Dies ermöglicht zugleich eine Verlängerung der Lebensdauer der mindestens einen Beleuchtungseinheit.
Die mindestens eine Beleuchtungseinheit kann in ihrer Lichtintensität von der Steuereinheit derart steuerbar sein, dass die Steuereinheit die Lichtintensität der mindestens einen Beleuchtungseinheit in Abhängigkeit der Drehzahl eines Ventilators steuert, der vor dem Lufteinlasskanal oder dem Luftauslasskanal angeordnet ist. Es kann somit zum einen die elektrische Leistungsaufnahme bedarfsgerecht gesteuert werden, zugleich kann vermieden werden, dass die mindestens eine Beleuchtungseinheit kontinuierlich, auch wenn nicht erforderlich, mit maximaler elektrischer Last, betrieben wird. Die mindestens eine Beleuchtungseinheit wird in ihrer Intensität in Abhängigkeit der das Gehäuse durchströmenden Luftmenge gesteuert, somit erfolgt eine bedarfsgerechte Ansteuerung. Dies ermöglicht außerdem eine Verlängerung der Lebensdauer der mindestens einen Beleuchtungseinheit, da diese nicht stets mit maximaler Last betrieben wird.

In einer vorteilhaften Ausgestaltung der Erfindung nach Patentanspruch 14 ist vorgesehen, dass eine erste Verschlussklappe angeordnet ist, die im Luftauslass angeordnet ist und eine an ihr horizontal angeordnete Platte aufweist, die im Übergangsbereich zwischen der Platte und der ersten Verschlussklappe einen Durchbruch aufweist, wobei die Platte im den Luftauslass verschließenden Zustand sich über den Ventilator erstreckt und damit den Luftstrom im Gehäuse gerichtet leitet.
Dadurch dass die Platte sich über den Ventilator erstreckt und vorzugsweise die gesamte Breite des Gehäuses aufnimmt, wird durch den Ventilator ein Luftstrom erzeugt. Der Ventilator saugt die Luft im Raumvolumenbereich an, drückt diese unterhalb der Platte gegen die erste Verschlussklappe. Durch den Durchbruch in der Platte gelangt der Luftstrom nach oben und strömt in Richtung der Luftreinigungseinheit und durchströmt diese.

In einer vorteilhaften Ausgestaltung der Erfindung nach Patentanspruch 15 ist vorgesehen, dass ein Bypass vorgesehen ist, der im oder am Luftauslass angeordnet ist und der Luft aus dem Gehäuse aus dem Innenraum des Fahrzeuges leitet. Dadurch kann die Luft aus dem Raumvolumenbereich direkt nach außen abgegeben werden. In einer vorteilhaften Ausgestaltung der Erfindung nach Patentanspruch 16 ist vorgesehen, dass im Gehäuse ein Reservoir zur Aufnahme von Wasser und/oder Kondenswasser vorgesehen ist. Das Reservoir dient zur Aufnahme von Kondenswasser. Da eine erhöhte Luftfeuchte bei der Selbstreinigung einen besseren Reinigungsvorgang gewährt, kann im Reservoir auch Wasser eingelagert werden. Das Reservoir ist vorteilhaft unterhalb des photokatalytisch wirksamen Oberflächenbereichs angeordnet, sodass von diesem abfallende Schadstoffe, wie z.B. Staubpartikel in das Reservoir fallen und dort vom Wasser gebunden werden.

In einer vorteilhaften Ausgestaltung der Erfindung nach Patentanspruch 17 ist vorgesehen, dass das Reservoir mit einer Abdeckklappe oder einer Abdeckung verschließbar ist.

In einer vorteilhaften Ausgestaltung der Erfindung nach Patentanspruch 18 ist vorgesehen, dass das Reservoir über eine Ableitung nach außerhalb des Innenraums des Fahrzeugs entleerbar oder von außerhalb des Innenraums des Fahrzeugs befüllbar ist. Über die Ableitung kann Wasser im Reservoir getauscht werden.

In einer vorteilhaften Ausgestaltung der Erfindung nach Patentanspruch 19 ist vorgesehen, dass die Abdeckklappe oder die Abdeckung ein Teil der zweiten Verschlussklappe ist.

Im Folgenden wird die erfindungsgemäße Luftreinigungsvorrichtung anhand konkreter Ausführungsbeispiele anhand von Figuren beschrieben. Die nachfolgende Beschreibung anhand der konkreten Ausführungsbeispiele stellt keine Limitierung der Erfindung auf eines dieser konkreten Ausführungsbeispiele dar.

### In den Figuren zeigt:

- FIG 1: einen schematischen Aufbau einer erfindungsgemäßen Luftreinigungsvorrichtung;
- FIG 2: eine perspektivische Ansicht einer Luftreinigungsvorrichtung und
- FIG 3: eine Ansicht einer erfindungsgemäßen Luftreinigungsvorrichtung mit den weiteren relevanten Bestandteilen;

In den Figuren sind gleiche Teile und/oder Komponenten mit gleichen Bezugszeichen versehen. Diese Teile und/oder Komponenten entsprechen im Wesentlichen einander, solange nichts anderen angegeben ist.

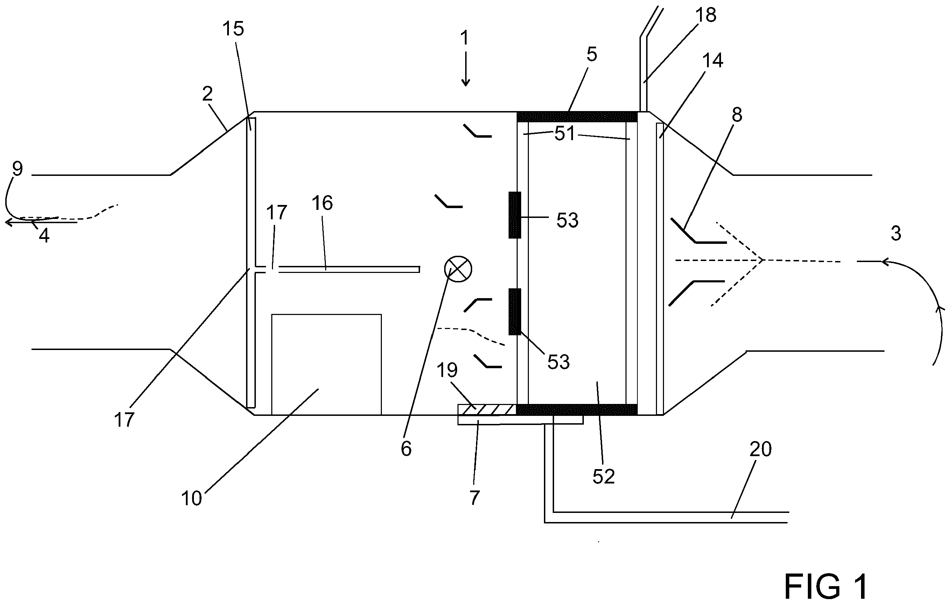

In FIG 1 ist ein Schnitt durch eine schematische Luftreinigungsvorrichtung 1 dargestellt. Die Luftreinigungsvorrichtung 1 besitzt ein Gehäuse 2. Die Form des Gehäuses 2 kann einen runden, ovalen, sechseckigen, n-eckigen oder, vorzugsweise, einen rechteckigen Querschnitt besitzen.

Als besonders vorteilhaft hat sich die Ausgestaltung des Querschnitts des Gehäuses 2 in einer rechteckigen Form ergeben. Das Gehäuse 2 ist quaderförmig.

Das Gehäuse 2 besteht vorzugsweise aus Kunststoff; in einer besonderen Ausgestaltung ist der gewählte Kunststoff ABS.

Das Gehäuse 2 weist an zwei sich gegenüberliegenden Enden eine Einlassöffnung in Form eines Lufteinlasskanals 3 und eine Auslassöffnung in Form eines Luftauslasskanals 4 für über den Lufteinlasskanal 3 dem Gehäuse 2 zugeführte Luft, auf.

Über den Lufteinlasskanal 3 wird zu reinigende Luft der Luftreinigungsvorrichtung 1 in deren Gehäuse 2 zugeführt; über den Luftauslasskanal 4 wird die in der Luftreinigungsvorrichtung 1 gereinigte Luft wieder aus dem Gehäuse 2 herausgeleitet.

Im Gehäuse 2 bildet sich ein Luftstrom 9. Der Luftstrom 9 durchläuft das Gehäuse 2 vom Lufteinlasskanal 3 zum Luftauslasskanal 4. Der Luftstrom ist im Gehäuse 2 gerichtet.

Im Gehäuse 2 ist eine Luftreinigungseinheit 5 angeordnet. Es können aber auch weitere Luftreinigungseinheiten im Gehäuse 2 angeordnet sein.

Die Luftreinigungseinheit 5 ist an einer der Innenwände des Gehäuses 2 angeordnet und mit der Innenwand form- oder kraftschlüssig verbindbar. Hierzu sind Klipse oder Halter vorgesehen, welche die Luftreinigungseinheit 5 an der Innenwand des Gehäuses 2 fixiert.

In Luftströmungsrichtung gesehen ist hinter der Luftreinigungseinheit 5 eine Beleuchtungseinheit 6 angeordnet. Es können auch mehrere Beleuchtungseinheiten 6 im Gehäuse 2 angeordnet werden.

Die Beleuchtungseinheit 6 ist nahezu mittig im Gehäuse 2 oder an der Innenwand des Gehäuses 2 so angeordnet, dass das von der Beleuchtungseinheit 6 emittierte Licht nahezu vollständig auf die Luftreinigungseinheit 5 gerichtet abgegeben wird. Vorzugsweise ist hinter der Beleuchtungseinheit 6 eine Reflektoreinheit angeordnet, welche auftreffendes Licht zurückwirft.

In einer vorteilhaften Ausgestaltung der Erfindung sind vor der Beleuchtungseinheit 6 Linsen angeordnet, die das Licht der Beleuchtungseinheit 6 auf die Luftreinigungseinheit 5 fokussieren.

In einer weiteren Ausgestaltung der Erfindung sind Prismen oder Spiegel an der Beleuchtungseinheit 6 angeordnet, welche verhindern, dass das abgegebene Licht der Beleuchtungseinheit 6 nicht auf die Luftreinigungseinheit 5 trifft.

In einer weiteren vorteilhaften Ausgestaltung der Erfindung ist das Innere des Gehäuses 2 zwischen der Luftreinigungseinheit 5 und der Beleuchtungseinheit 6 weiß lackiert oder mit einer lichtreflektierenden Oberflächenbeschichtung versehen.

Die Luftreinigungseinheit 5 besitzt mindestens einen photokatalytisch wirksamen Bereich 53. In der Ausgestaltung nach FIG 1 sind bei der Luftreinigungseinheit 5 zwei derartige Bereiche 53 vorhanden.

Die photokatalytisch wirksamen Bereiche 53 bestehen in der konkreten Ausgestaltung nach FIG 1 aus Titandioxid - TiO2 - oder sind mit Titanoxidionen - TiO2-Ionen - dotiert oder versetzt oder durchsetzt. Es können jedoch auch andere photokatalytisch aktive Materialien als TiO2 eingesetzt werden, insbesondere die bereits genannten Materialien, insbesondere Metalloxid oder Mischoxid von aus CuO, Co₃O₄, CoOₓ, NiO, MnOₓ, MnO₂, MoO₃, ZnO, Fe₂O₃, WO₃, CeO₂, TiO₂, Al₂O₃, V₂O₃, ZrO₂, HfO₂, Dy₂O₃, Cr₂O₃ LiNbO₃ und/oder Nb₂O₅. Es können aber auch andere photokatalytisch wirksame Stoffe und/oder Verbindungen eingesetzt werden. Die vorgenannte Aufzählung ist nicht abschließend.

Die Luftreinigungseinheit 5 bestehen aus einem Rahmen und einem Vlies 51, das den Rahmen umspannt, zumindest partiell, und einen Innenraum angrenzt, in dem Aktivkohle angeordnet oder der mit Aktivkohle versetzt ist. Dieser mit Aktivkohle durchsetzter bzw. versetzte bzw. versetzbare Bereich ist mit dem Bezugszeichen 52 versehen.

Die Beleuchtungseinrichtung 6 ist auf die photokatalytisch wirksamen Bereiche 53 abgestimmt. Die Beleuchtungseinheit 6 gibt UV-Licht oder sichtbares Licht mit einer voreinstellbaren oder voreingestellten Wellenlänge ab. Die Photonen mit der entsprechenden Wellenlänge des UV-Lichts oder sichtbaren Lichts der Beleuchtungseinrichtung 6 lösen beim Auftreffen auf den jeweiligen photokatalytisch wirksamen Bereich 53 eine photochemischen Reaktion im Titanoxid oder den anderen vorgenenannten Materialien aus, die dazu führt, dass Geruchsstoffpartikel und/oder Schadpartikel in der Luft umgewandelt bzw. zerstört werden. Die auf die photokatalytisch wirksamen Bereiche 53 auftreffenden Geruchsstoffpartikel und/oder Schadpartikel werden durch den photochemischen Prozess zerstört oder umgewandelt und damit wird die zugeführte Luft gereinigt.

Bei der Beleuchtungseinrichtung 6 handelt es sich vorzugsweise um mindestens eine Leuchtdiode, vorzugsweise eine UV-Leuchtdiode. UV hat die Bedeutung von ultraviolett.

In einer vorteilhaften Ausgestaltung der Erfindung sind mehrere UV-Leuchtdioden angeordnet und bilden die Beleuchtungseinrichtung 6. Die Leuchtdioden sind zueinander in einer Reihe, äquidistant zueinander angeordnet. Es sind dann mehrere Reihen von UV-Leuchtdioden parallel nebeneinander und in gleichem Abstand zueinander anordenbar.

Die Beleuchtungseinrichtung 6 wird über eine nicht in FIG 1 dargestellten Steuereinheit angesteuert.

Im Gehäuse 2 sind Luftleitelemente 8 angeordnet. Diese dienen dazu, den Luftstrom 9 im Gehäuse 2 auf die Luftreinigungseinheit 5 und die photokatalytisch wirksamen Bereiche 53 zu lenken, sodass ein möglichst großer Teil des Luftstroms 9 mit den Schadpartikeln und/oder Schadstoffpartikeln zu den photokatalytisch wirksamen Bereichen 53 gelangt, um dort photokatalytisch zu reagieren. Um eine möglichst gute Luftumströmung der photokatalytisch wirksamen Bereiche 53 der Luftreinigungseinheit 5 zu erreichen, und zugleich nicht die Lichtabgabe der Beleuchtungseinheit 6 auf die Luftreinigungseinheiten 5 zu beeinträchtigen, sind die Luftleitelement 8 vor und nach der Beleuchtungseinheit 6 angeordnet. In einer vorteilhaften Ausgestaltung sind die Luftleitelemente 8 mit einer mit UV-Licht reflektierenden Beschichtung versehen.

Durch die Luftreinigungseinheit 5 und deren photokatalytisch wirksamen Bereiche 53 wird, bei Auftreffen von UV-Licht, abgestrahlt von der Beleuchtungseinheit 6, entsprechend die das Gehäuse 2 durchströmende Luft gereinigt.

Die Luftreinigungseinheit 5 besitzt einen Rahmen, der von einem Vlies 51 umspannt ist und damit einen Innenraum 52 begrenzt. In den Innenraum 52 sind Aktivkohle oder ein Granulat aus Aktivkohle und/oder ein Gemisch aus Aktivkohle und ein Granulat aus mit Titanoxid dotiertem oder versetztem Granulat eingefüllt. Es können auch die bereits vorgenannten Materialien wie Metalloxid oder Mischoxid von aus CuO, Co₃O₄, CoOₓ, NiO, MnOₓ, MnO₂, MoO₃, ZnO, Fe₂O₃, WO₃, CeO₂, TiO₂, Al₂O₃, V₂O₃, ZrO₂, HfO₂, Dy₂O₃, Cr₂O₃ LiNbO₃ und/oder Nb₂O₅ zum Einsatz kommen.

Als Träger des Granulats für die Titanoxidionen bzw. die vorgenannten Materialien kommen Eisenpartikel oder Kunststoffpartikel zur Anwendung.

Auf der der Beleuchtungseinheit 6 zugeordneten bzw. zugewandten Seite der Luftreinigungseinheit 5 ist das dortig angeordnete Vlies 51 mit einem fotokatalytisch wirksamen Oberflächenbereich 53 ausgestattet.

Der fotokatalytische und fotokatalytisch wirksame Oberflächenbereich 53 ist mit Titanodxidionen versetzt bzw. durchsetzt. Durch auftreffen von UV-Licht oder Licht auf diese Bereiche 53 wird der photokatalytische Prozess in Gang gesetzt. Schadstoffe, die mit dem Luftstrom 9 vorbeigeführt werden, werden entsprechend reduziert, zersetzt oder aufgelöst.

Die fotokatalytisch wirksamen Oberflächenbereiche 53 sind stets in Luftströmungsrichtung hinter dem Bereich 52 mit Aktivkohle angeordnet. Damit wird verhindert, dass die fotokatalytisch wirksamen Oberflächenbereiche 53 sich über die Zeit hin zusetzen und inaktiv werden.

Das Vlies 51 ist zumindest partiell, zur Ausbildung der photokatalytisch wirksamen Oberflächenbereiche 53 mit Titanoxidionen oder einem der vorgenannten Materialien dotiert oder versetzt oder aber es sind an diesen Stellen Fäden, oder Fasern, die mit Titanoxidionen oder einem der vorgenannten Materialien oder einem Materialmix aus diesen Materialien dotiert oder versetzt sind, in das Vlies 51 eingebracht, eingewebt oder mit diesem verbunden. Alternativ kann auch die Dotierung mit Titanoxidionen bzw. oder einem der vorgenannten Materialien oder einem Materialmix aus diesen Materialien oder die Ausbildung dieser photokatalytisch wirksamen Bereiche 53 erfolgen, in dem das Vlies 51 mit einer Flüssigkeit, in der Titanoxidionen oder eines der vorgenannten Materialien oder einen Materialmix aus diesen Materialien enthalten bzw. gelöst sind, getaucht wird oder mit dieser besprüht bzw. benetzt wird.

Das Vlies 51 ist in der Weise ausgestaltet, dass es sogleich als Filter dient. Hierbei kann durchaus die Struktur eines HEPA-Filters als Vorlage für das Vlies 51 dienen, sodass zusätzlich eine Reinigungswirkung von Grobpartikeln, die mit dem Luftstrom 9 angesaugt werden, erfolgt. Hierbei ist es vorteilhaft, dass das Vlies 51 beidseitig an der Luftreinigungseinheit 5 angeordnet ist, sodass bereits der eintretende Luftstrom 9 durch das Vlies 51 vorgereinigt wird und Grobpartikel aus dem Luftstrom 9 gefiltert werden. Hierdurch wird auch eine Verschmutzung der photokatalytisch wirksamen Bereiche 53 vermieden.

Im Weiteren ist es vorteilhaft, die Oberfläche des Vlieses 51 und damit die photokatalytisch wirksamen Bereiche 53 großflächig auszugestalten. Daher hat es sich als vorteilhaft erwiesen die Oberfläche durch Faltung oder Verformung, beispielsweise durch Ausbildung einer Wellenform zu vergrößern. Im Weiteren ist es auch möglich diese Oberfläche mit kegel-, falten-, zylinder-, kegelstumpf-, pyramidenstumpf-, kugel-, oder halbkugelgeometrischen Ausformungen auszugestalten.

Ein weiterer Vorteil der Erfindung ist darin zu sehen, dass durch die photokatalytisch wirksamen Bereiche 53 nicht nur die Luft, sondern auch die angrenzenden Bereiche mitgereinigt werden. Somit kann zugleich auch eine Reinigungswirkung auf das gesamte Vlies 51, als auch auf die dahinter angeordneten Bereiche mit Aktivkohle 52 erfolgen.

Besonders vorteilhaft ist es, wie bereits ausgeführt, wenn das Titanoxid bzw. die Titanoxidionen oder einem der vorgenannten Materialien oder einem Materialmix aus diesen Materialien auch in Form von Granulat oder in anderer Form in den Aktivkohlebereich 52 direkt eingebunden werden. Dadurch wird, wenn die Titanoxidionen bzw. die vorgenannten Materialien oder ein Materialmix aus diesen Materialien über den photokatalytischen Prozess aktiviert werden, auch die Aktivkohle selbst entsprechend gereinigt.

Es ist vorgesehen, dass die Luftreinigungsvorrichtung 1 einen Selbstreinigungsprozess besitzt. Hierzu wird das Gehäuse 2 auf der Lufteinlassseite und der Luftauslassseite jeweils von einer Verschlussklappe 14 verschlossen. In einer vorteilhaften Ausgestaltung der Erfindung wird anstelle einer Klappe eine Membran eingesetzt.

Es entsteht damit im Gehäuse 2 ein abgegrenzter Raumvolumenbereich. Dieser Bereich wird nun über die Beleuchtungseinheit 6, die mit maximal zulässiger Energieangabe betrieben wird, aufgeheizt. Außerdem ist ein Ventilator 10 im abgegrenzten Raumvolumenbereich angeordnet, der einen Luftstrom im abgegrenzten Raumvolumenbereich erzeugt. Durch die Aufheizung wird der Selbstreinigungseffekt der Aktivkohle aktiviert. Durch den Luftstrom wird dieser Prozess verstärkt.

Durch die Aufheizung des über die Verschlussklappen 14, 15 angegrenzten Raumvolumenbereichs des Gehäuses 2 über die aktivierte Beleuchtungseinheit 6 wird in der Aktivkohle gebundenes Wasser ausgegast. Die hierdurch entstehende erhöhte Luftfeuchtigkeit trägt zur Verbesserung der Reinigungswirkung im Rahmen der Selbstreinigung bei. Zugleich wird durch die Temperaturerhöhung, die UV-Bestrahlung und den über den Ventilator 10 erzeugten Luftstrom der Luftstrom und die gesamten weiteren Komponenten und Bereiche 51, 52, 53 erhitzt. Hierdurch wird eine Schadstoffdesorption aus den Komponenten und Bereichen 51, 52, 53 erreicht und die Aktivkohle getrocknet. Nach einem vordefinierbaren Zeitraum werden die Verschlussklappen 14, 15 geöffnet und die desorptierten Stoffe entweichen. Die Luftreinigungsvorrichtung hat den Selbstreinigungsprozess abgeschlossen. Es ist insoweit vorgesehen, dass die Luft mit den desorptierten Stoffen über einen Bypass 18 außerhalb des Innenraums des Fahrzeuges ausleitbar ist.

Die Verschlussklappe 15 ist im Luftauslass 4 angeordnet. An der Verschlussklappe 15 ist eine horizontal angeordnete Platte 16 vorhanden, die rechtwinklig von der Verschlussklappe 15 absteht. Im Übergangsbereich zwischen der Platte 16 und der Verschlussklappe 15 ist einen Durchbruch 17 vorhanden. Im den Luftauslass 4 verschließenden Zustand der Verschlussklappe 15 erstreckt sich die Platte 16 über den Ventilator 10. Es bildet sich ein Luftkanal aus, gebildet von dem Bereich im Gehäuse 2 oberhalb der Platte 16, den Durchbruch 17 und unterhalb der Platte 16, der durch den Ventilator 10 erzeugt wird. Vorzugsweise nimmt die Platte 16 die gesamte Breite des Gehäuses 2 auf. Der Ventilator 10 saugt die Luft im abgeschlossenen Raumvolumenbereich des Gehäuses 2 an, drückt diese unterhalb der Platte 16 gegen die Verschlussklappe 15 und durch den Durchbruch 17. Oberhalb der Platte 16 strömt die Luft dann in Richtung der Luftreinigungseinheit 5.

Es ist ein Bypass 18 vorgesehen, der im oder am Lufteinlasskanal 3 angebunden ist. Alternativ kann dieser aber auch im bzw. am Luftauslasskanal 4 angeordnet werden. Über den Bypass 18 kann Luft nach dem Selbstreinigungsprozess aus dem abgegrenzten Raumvolumenbereich nach außerhalb des Innenraums des Fahrzeuges geleitet werden.

Im Gehäuse 2 ist ein Reservoir 7 zur Aufnahme von Wasser und/oder Kondenswasser vorgesehen. Das Reservoir 7 dient zur Aufnahme von Kondenswasser. Da eine erhöhte Luftfeuchte bei der Selbstreinigung einen besseren Reinigungsvorgang gewährt, kann im Reservoir 7 auch Wasser eingelagert werden, das beim Selbstreinigungsvorgang zur Erhöhung der Luftfeuchte dient.

Das Reservoir 7 ist mit einer Abdeckklappe 19 verschließbar. Die Abdeckklappe 19 ist in einer vorteilhaften Ausgestaltung ein Teil der Verschlussklappe 14.

Es ist eine Ableitung 20 aus dem Reservoir 7 vorgesehen. Über die Ableitung 20 kann nach außerhalb des Innenraums des Fahrzeugs das Reservoir entleert werden. Es kann aber auch das Reservoir 7 von außerhalb des Innenraums des Fahrzeugs über die Ableitung 20 befüllt werden; es kann via der Ableitung 20 Wasser im Reservoir 7 getauscht werden.

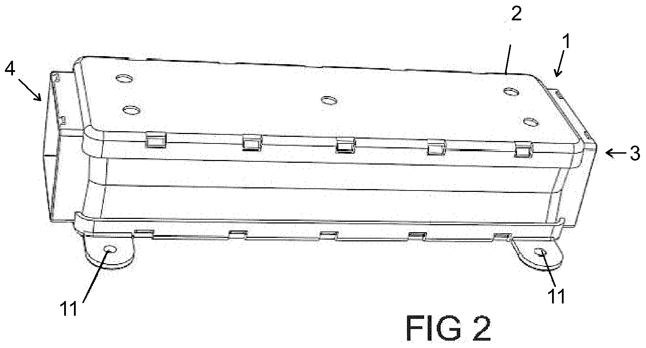

In FIG 2 ist das Gehäuse 2 der Luftreinigungsvorrichtung 1 perspektivisch dargestellt. Das Gehäuse 2 besteht aus mehreren Einzelteilen, die sich zu dem Gehäuse 2 zusammensetzen lassen. Das Gehäuse 2 ist quaderförmig ausgestaltet und weist zur einen Seite eine Verjüngung auf, die in den Lufteinlasskanal 3 übergeht. Auf der dem Lufteinlasskanal 3 gegenüberliegenden Seite des Gehäuses 2 ist eine weitere entsprechende Verjüngung vorgesehen, die die den Luftauslasskanal 4 bildet. Die zu reinigende Luft wird dem Kraftfahrzeuginnenraum entnommen, insbesondere aus diesem abgesaugt, und über den Lufteinlasskanal 3 dem Gehäuse 2 und damit der Luftreinigungsvorrichtung 1 zugeführt. Im Gehäuse 2 erfolgt dann die Reinigung der Luft, analog wie bei FIG 1 beschrieben, und die gereinigte Luft wird dann über den Luftauslasskanal 4 ausgeleitet und dann dem Fahrzeuginnenraum wieder zugeführt.

Im Weiteren sind am Gehäuse Aufnahmen 11 vorhanden, die fest mit dem Gehäuse 2 verbunden sind. Diese Aufnahmen 11 dienen dazu, die Luftreinigungsvorrichtung 1 in einem Kraftfahrzeug an eine vorgesehene Stelle befestigen zu können. Hierzu ist eine Klemmung oder eine Verschraubung vorgesehen.

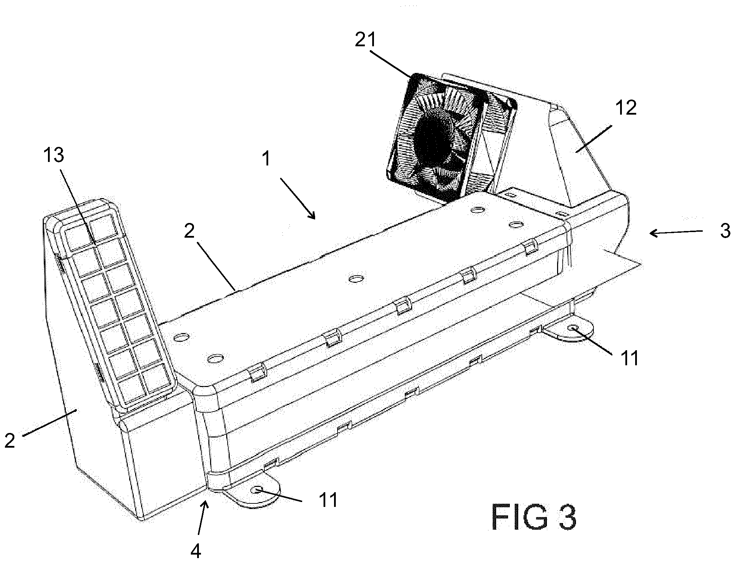

In FIG 3 ist die Luftreinigungsvorrichtung 1 mit dem Gehäuse 2 dargestellt. Es ist am Lufteinlasskanal 3 ein Luftzuführungskanal 12 angeordnet, an dessen Eingang ein Ventilator 21 angeordnet ist, der Luft aus dem Fahrzeuginnenraum ansaugt und durch den Luftzuführungskanal 12 und über den Lufteinlasskanal 3 in das Gehäuse 2 der Luftreinigungsvorrichtung 1 bläst, vorhanden. Der Luftzuführungskanal 12 ist auf die Verjüngung des Lufteinlasskanals 3 aufgesteckt und ist formschlüssig mit diesem verbunden.

In einer besonderen Ausgestaltung der Erfindung ist der Luftzuführungskanal 12 mittels einer Schnappvorrichtung mit dem Lufteinlasskanal 3 oder dem Gehäuse 2 verbindbar. Auf der Seite der Luftausgangsöffnung 4 ist ein Luftführungskanal 13 angeordnet, der die ausströmende Luft aus der Luftausgangsöffnung 4 aufnimmt, kanalisiert und entsprechend an den Innenraum eines Kraftfahrzeuges, aus dem mittels des Ventilators 21 die Luft abgesaugt worden ist, wieder zugeführt.

In einer weiteren vorteilhaften Ausgestaltung der Erfindung ist vorgesehen, dass in dem Luftführungskanal 12 mindestens eine weitere Luftfiltereinheiten, wie beispielsweise ein HEPA-Filter oder ein anderer Luftfilter, angeordnet ist.

Das Gehäuse 2 besitzt einen Gehäuseboden und einen Gehäusedeckel, die gemeinsam das Gehäuse 2 bilden. Am Gehäuseboden sind die Aufnahmen 11 angeordnet. Seitlich außenseitig weisen sowohl der Gehäuseboden als auch der Gehäusedeckel, mehrere Einbuchtungen auf, in welche korrespondierende Nasen an den Gehäuseseitenteilen einschnappen und so den Gehäuseboden und den Gehäusedeckel über die Gehäuseseitenteile verbinden.

Auf der Innenseite des Gehäusedeckels und der Innenseite des Gehäusebodens ist die Luftreinigungseinheit 5 angeordnet bzw. dort fixiert. Die Luftreinigungseinheit 5 ist form- und/oder kraftschlüssig mit dem Gehäuseboden oder dem Gehäusedeckel verbunden. Diese Verbindung erfolgt beispielsweise über eine Verklipsung, Verklebung oder Verschraubung.

Bei der Beleuchtungseinheit 6 handelt es sich um eine oder mehrere Leuchtdioden, die ultraviolettes Licht emittieren, das vorzugsweise auf das Titandioxid und dessen dadurch erzeugbaren photokatalytischen Wirkung abgestimmt ist, und eine Wellenlänge im Bereich zwischen 250 und 400 Nanometer besitzt, vorzugsweise im Bereich um die 367 Nanometer. Bei Einsatz von dotiertem TiO2 kann sichtbares Licht im Bereich von 400 bis 500 Nanometer zum Einsatz kommen, vorzugsweise mit 455 nm Wellenlänge.

### Bezugszeichenliste

- 1: Luftreinigungsvorrichtung
- 2: Gehäuse
- 3: Lufteinlasskanal
- 4: Luftauslasskanal
- 5: Luftreinigungseinheit
- 51: Vlies
- 52: mit Aktivkohle durchsetzter Bereich
- 53: photokatalytisch wirksamer Oberflächenbereich
- 6: Beleuchtungseinheit
- 7: Reservoir
- 8: Luftleitelemente
- 9: Luftstrom
- 10, 21: Ventilator
- 11: Aufnahme(n)
- 12: Luftzuführungskanal
- 13: Luftführungskanal
- 14, 15: Verschlussklappe
- 16: Platte
- 17: Durchbruch
- 18: Bypass
- 19: Abdeckklappe
- 20: Ableitung

## Patentansprüche

1. Luftreinigungsvorrichtung (1), bestehend aus einem Gehäuse (2), das mindestens eine Eingangsöffnung (3) zur Zuführung eines Luftstroms (9) und mindestens eine Ausgangsöffnung (4) zur Ausleitung des über die Eingangsöffnung (3) zugeführten Luftstroms (9) besitzt, wobei im Gehäuse (2) mindestens eine Luftreinigungseinheit (5) und mindestens eine Beleuchtungseinheit (6) angeordnet sind, wobei die mindestens eine Luftreinigungseinheit (5) im Wesentlichen die Form des Querschnitts des Gehäuses (2) aufnimmt, im Gehäuse (2) die mindestens eine Beleuchtungseinheit (6) der mindestens einen Luftreinigungseinheit (5) gegenüberliegend angeordnet ist, die mindestens eine Luftreinigungseinheit (5) luftdurchlässig ausgestaltet ist und mindestens einen mit Aktivkohle durchsetzten Bereich (52) aufweist, der zumindest partiell von einem Vlies (51) umgeben ist, wobei auf der der Beleuchtungseinheit (6) zugewandten Seite der mindestens einen Luftreinigungseinheit (5) mindestens ein photokatalytisch wirksamen Oberflächenbereich (53) im oder am Vlies (51) vorhanden ist, der Luftstrom (9) im Gehäuse (2) zumindest partiell den mindestens einen photokatalytisch wirksamen Oberflächenbereich (53) und den mit Aktivkohle durchsetzen Flächenbereich (52) umströmt bzw. durchströmt, wobei der mindestens eine photokatalytisch wirksame Oberflächenbereich (53) der mindestens einen Luftreinigungseinheit (5) von der mindestens einen Beleuchtungseinheit (6) mit Licht anstrahlbar ist, **dadurch gekennzeichnet, dass** die mindestens eine Eingangsöffnung (3) zur Zuführung eines Luftstroms (9) und die mindestens eine Ausgangsöffnung (4) zur Ausleitung des über die Eingangsöffnung (3) zugeführten Luftstroms (9) mittels jeweils einer Verschlussklappe (14, 15) schließbar sind und im geschlossenen Zustand der beiden Verschlussklappen (14, 15) im Gehäuse (2) einen abgegrenzten Raumvolumenbereich begrenzen, ein Ventilator (10) im Gehäuse (2) im abgegrenzten Volumenbereich angeordnet ist, der im abgegrenzten Volumenbereich des Gehäuses (2) einen Luststrom erzeugt, wobei der erzeugte Luftstrom den mit Aktivkohle durchsetzten Bereich (52) durchströmt, und die mindestens eine Beleuchtungseinheit (6) den abgegrenzten Volumenbereich des Gehäuses (2) und damit den Luftstrom und den mit Aktivkohle durchsetzten Bereich (52) erhitzt und somit eine Selbstreinigung des mit Aktivkohle durchsetzten Bereichs (52) und/oder des Vlieses (51) und/oder des photokatalytisch wirksamen Oberflächenbereich (53) erfolgt.

2. Luftreinigungsvorrichtung (1) nach Patentanspruch 1, **dadurch gekennzeichnet, dass** der mindestens eine photokatalytisch wirksame Oberflächenbereich (53) zumindest teilweise mit einem Metalloxid oder einem Mischoxid beschichtet oder mit einem Metalloxid oder einem Mischoxid - versetzt oder durchsetzt ist und dass zusätzlich Heizdrähte integriert sind, die bei der Selbstreinigung zur schnellen Erhitzung beitragen und/oder wobei das Metalloxid ein Oxid aus einem Übergangsmetall ist.

3. Luftreinigungsvorrichtung (1) nach dem vorangehenden Patentanspruch 2, **dadurch gekennzeichnet, dass** das Metalloxid oder Mischoxid von CuO, Co₃O₄, CoOₓ, NiO, MnOₓ, MnO₂, MoO₃, ZnO, Fe₂O₃, WO₃, CeO₂, TiO₂, Al₂O₃, V₂O₃, ZrO₂, HfO₂, Dy₂O₃, Cr₂O₃ und/oder Nb₂O₅.

4. Luftreinigungsvorrichtung (1) nach Patentanspruch 1, **dadurch gekennzeichnet, dass** die der photokatalytisch wirksamen Oberflächenbereich (53) derart abgeordnet ist, dass diese nach unten in Richtung des Bodens des Gehäuses (2) geneigt ist.

5. Luftreinigungsvorrichtung (1) nach Patentanspruch 1, **dadurch gekennzeichnet, dass** die mindestens eine Luftreinigungseinheit (5) an einer der Innenwände des Gehäuses (2) fixierbar ist.

6. Luftreinigungsvorrichtung (1) nach einem der vorangehenden Patentansprüche 1 bis 5, **dadurch gekennzeichnet, dass** die mindestens eine Beleuchtungseinheit (6) im Gehäuse (2) zwischen dem mindestens einen photokatalytisch wirksamen Oberflächenbereich (53) und der Luftaustrittsöffnung (4) angeordnet ist, und wobei nur die Innenwände des Gehäuses (2) im Bereich zwischen der mindestens einen Luftreinigungseinheit (5) und der mindestens einen Beleuchtungseinheit (6) mit einem lichtreflektierenden Material, insbesondere einem UV-Licht reflektierenden Material, beschichtet sind.

7. Luftreinigungsvorrichtung (1) nach einem der vorangehenden Patentansprüche, **dadurch gekennzeichnet, dass** vor der mindestens einen Beleuchtungseinheit (6) Linsen angeordnet sind, die das von der mindestens einen Beleuchtungseinheit (6) emittierte Licht auf die mindestens eine Luftreinigungseinheit (5) fokussieren oder hinter und/oder neben der mindestens einen Beleuchtungseinheit (6) Lichtreflektoren oder Licht reflektierende Bereiche angeordnet sind, welche das von der mindestens einen Beleuchtungseinheit (6) emittierte Licht in Richtung der mindestens einen Luftreinigungseinheit (5) reflektieren.

8. Luftreinigungsvorrichtung (1) nach dem vorangehenden Patentanspruch 7, **dadurch gekennzeichnet, dass** das lichtreflecktierende Material ein weißes Lack oder ein insbesondere UV-Licht reflektierendes Material ist.

9. Luftreinigungsvorrichtung (1) nach einem der vorangehenden Patentansprüche, **dadurch gekennzeichnet, dass** im Gehäuse (2) Luftleitelemente (8) angeordnet sind, welche den Luftstrom (9) diffus im Gehäuse (2) ablenken, sodass der Luftstrom (9) nahezu gleichmäßig auf die gesamte Oberfläche des Vlieses (51) der mindestens einen Luftreinigungseinheit (5) trifft und diese durchströmt oder der Luftstrom (9) größtenteils auf den mindestens einen photokatalytisch wirksamen Oberflächenbereich (53) gerichtet ist und diesen umströmt bzw. durchströmt.

10. Luftreinigungsvorrichtung (1) nach einem der vorangehenden Patentansprüche, **dadurch gekennzeichnet, dass** die mindestens eine Luftreinigungseinheit (5) aus einem Rahmen aus Kunststoff oder Metall besteht, welcher vom Vlies (51) zumindest an einer Seite umschlossen ist und hinter dem Vlies (51) ein Aufnahmebereich für Granulatstoffe vorhanden ist, wobei dieser Aufnahmebereich mit Aktivkohle oder einem Gemisch aus Aktivkohle und mit Titandioxid - TiO2 - oder Metalloxid oder Mischoxid von CuO, Co₃O₄, CoOₓ, NiO, MnOₓ, MnO₂, MoO₃, ZnO, Fe₂O₃, WO₃, CeO₂, TiO₂, Al₂O₃, V₂O₃, ZrO₂, HfO₂, Dy₂O₃, Cr₂O₃ und/oder Nb₂O₅ beschichtetem oder mit Titandioxidionen - TiO2-Ionen - und/oder Metalloxid-Ionen oder Mischoxid-Ionen von CuO, Co₃O₄, CoOₓ, NiO, MnOₓ, MnO₂, MoO₃, ZnO, Fe₂O₃, WO₃, CeO₂, TiO₂, Al₂O₃, V₂O₃, ZrO₂, HfO₂, Dy₂O₃, Cr₂O₃ und/oder Nb₂O₅ versetztem Granulat befüllt ist.

11. Luftreinigungsvorrichtung (1) nach einem der vorangehenden Patentansprüche, **dadurch gekennzeichnet, dass** zumindest partiell Fäden oder Fasern in das Vlies (51) eingearbeitet oder eingebracht sind, die mit Titandioxidionen - TiO2-Ionen oder Metalloxid-Ionen oder Mischoxid-Ionen von CuO, Co₃O₄, CoOₓ, NiO, MnOₓ, MnO₂, MoO₃, ZnO, Fe₂O₃, WO₃, CeO₂, TiO₂, Al₂O₃, V₂O₃,ZrO₂, HfO₂, Dy₂O₃, Cr₂O₃ und/oder Nb₂O₅ dotiert sind, welche den mindestens einen photokatalytisch wirksamen Oberflächenbereich (53) ausbildet oder dass Bereiche (53) des Vlieses (51) mit Titandioxidionen - TiO2-Ionen oder Metalloxid-Ionen oder Mischoxid-Ionen von CuO, Co₃O₄, CoOₓ, NiO, MnOₓ, MnO₂, MoO₃, ZnO, Fe₂O₃, WO₃, CeO₂, TiO₂, Al₂O₃, V₂O₃, ZrO₂, HfO₂, Dy₂O₃, Cr₂O₃ und/oder Nb₂O₅ dotiert sind.

12. Luftreinigungsvorrichtung (1) nach einem der vorangehenden Patentansprüche, **dadurch gekennzeichnet, dass** die Oberfläche der mindestens einen Luftreinigungseinheit (5) mit dem Vlies (51) zumindest an der den mindestens einen photokatalytisch wirksamen Oberflächenbereich (53) aufweisenden Seite wellenförmig ausgestaltet ist oder kegel-, falten-, zylinder-, kegelstumpf-, pyramidenstumpf-, kugel- oder halbkugelgeometrische Ausformungen besitzt.

13. Luftreinigungsvorrichtung (1) nach einem der vorangehenden Patentansprüche, **dadurch gekennzeichnet, dass** die mindestens eine Beleuchtungseinheit (6) in ihrer Lichtintensität steuerbar ist und bei der Selbstreinigung mit maximaler Intensität angesteuert ist.

14. Luftreinigungsvorrichtung (1) nach einem der vorangehenden Patentansprüche, **dadurch gekennzeichnet, dass** erste Verschlussklappe (15), die im Luftauslass (4) angeordnet ist, eine horizontal angeordnete Platte (16) aufweist, die im Übergangsbereich zwischen Platte (16) und Verschlussklappe (15) eine Durchbruch (17) aufweist, wobei die Platte (16) im den Luftauslass (5) verschließenden Zustand sich über den Ventilator (10) erstreckt und damit den Luftstrom im Gehäuse (2) gerichtet leitet.

15. Luftreinigungsvorrichtung (1) nach einem der vorangehenden Patentansprüche, **dadurch gekennzeichnet, dass** ein Bypass (18) vorgesehen ist, der im oder am Luftauslass (5) angeordnet ist und der Luft aus dem Gehäuse (2) aus dem Innenraum des Fahrzeuges leitet.

16. Luftreinigungsvorrichtung (1) nach einem der vorangehenden Patentansprüche, **dadurch gekennzeichnet, dass** im Gehäuse (2) ein Reservoir (7) zur Aufnahme von Wasser und/oder Kondenswasser vorgesehen ist.

17. Luftreinigungsvorrichtung (1) nach dem vorangehenden Patentanspruch 16, **dadurch gekennzeichnet, dass** das Reservoir (7) mit einer Abdeckklappe (19) oder einer Abdeckung verschließbar ist.

18. Luftreinigungsvorrichtung (1) nach dem vorangehenden Patentanspruch 16 oder 17, **dadurch gekennzeichnet, dass** das Reservoir (7) über eine Ableitung (20) nach außerhalb des Innenraums des Fahrzeugs entleerbar oder von außerhalb des Innenraums des Fahrzeugs befüllbar ist.

19. Luftreinigungsvorrichtung (1) nach dem vorangehenden Patentanspruch 17, **dadurch gekennzeichnet, dass** die Abdeckklappe (19) oder die Abdeckung ein Teil der zweiten Verschlussklappe (14) ist.
